(19) Europäisches Patentamt
European Patent Office
Office européen des brevets

(11) Veröffentlichungsnummer: **0 444 448 A2**

(12) **EUROPÄISCHE PATENTANMELDUNG**

(21) Anmeldenummer: **91101643.4**

(22) Anmeldetag: **07.02.91**

(51) Int. Cl.[5]: **C07C 43/225**, C09K 19/30, C07C 255/50

(30) Priorität: **15.02.90 EP 90102937**
**15.02.90 DE 4004650**

(43) Veröffentlichungstag der Anmeldung:
**04.09.91 Patentblatt 91/36**

(84) Benannte Vertragsstaaten:
**DE GB**

(71) Anmelder: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Reiffenrath, Volker**
**Jahnstrasse 18**
**W-6101 Rossdorf(DE)**
Erfinder: **Hittich, Reinhard, Dr.**
**Am Kirchberg 11**
**W-6101 Modautal 1(DE)**
Erfinder: **Rieger, Bernhard, App. B511**
**WACORE-Tanagawa-gakuen**
**2834 Ootadaiva Naramachi Midari-ku,**
**Yokohame-shi**
**Kanagawa pref. 227(JP)**

(54) **Phenylcyclohexylethane und flüssigkristallines Medium.**

(57) Phenylcyclohexylethane der Formel I

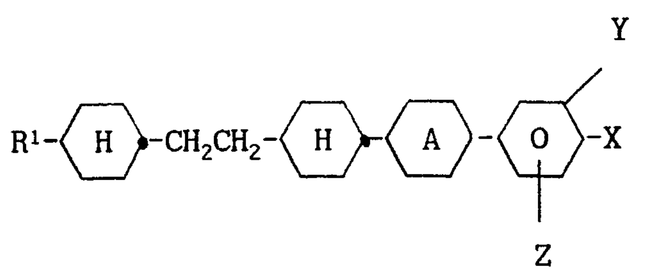

I

worin $R^1$ Alkyl oder Oxaalkyl mit bis zu 9 C-Atomen, Ring A trans-1,4-Cyclohexylen, 1,4-Phenylen, 3-Fluor1,4-phenylen oder 3,5-Difluor-1,4-phenylen, X eine der Bedeutungen von $R^1$, F, Cl, $-CF_3$, -CN, $-OCF_3$ oder $-OCHF_2$ und Y und Z jeweils unabhängig voneinander H oder F bedeuten,
können als Komponenten flüssigkristalliner Medien verwendet werden.

EP 0 444 448 A2

Die Erfindung betrifft Phenylcyclohexylethane der Formel I

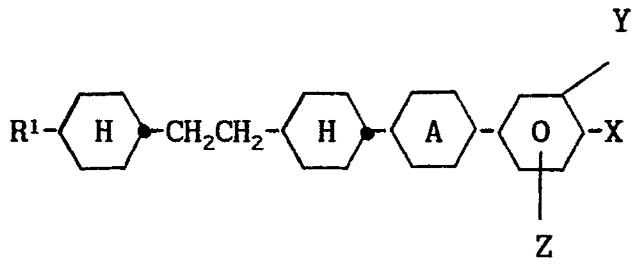

I

worin $R^1$ Alkyl oder Oxaalkyl mit bis zu 9 C-Atomen, Ring A trans-1,4-Cyclohexylen, 1,4-Phenylen, 3-Fluor1,4-phenylen oder 3,5-Difluor-1,4-phenylen, X eine der Bedeutungen von $R^1$, F, Cl, $-CF_3$, -CN, $-OCF_3$ oder $-OCHF_2$ und Y und Z jeweils unabhängig voneinander H oder F bedeuten.

Aus der EP-OS 0 272 580 sind Flüssigkristalle der Formel B bekannt

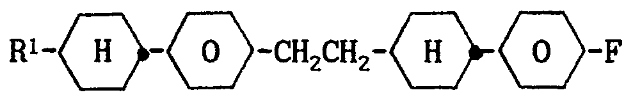

B

worin $R^1$ $C_2H_5$ oder $C_4H_7$ bedeutet.

Die Verbindungen B zeichnen sich nur durch eine moderate dielektrische Anisotropie aus, die in Mischungen oft zu relativ hohen Schwellenspannungen führt. Die Verbindungen B zeichnen sich ferner durch recht hohe Schmelzpunkte und relativ ungünstige Mischbarkeit mit anderen Flüssig˜ kristallverbindungen aus.

Diese Verbindungen der Formel I können wie ähnliche, z.B. aus der DE-OS 32 11 601 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung.

Insbesondere bei Anzeigen vom Supertwisttyp (wie z.B. STN, SBE, OMI) mit Verdrillungswinkeln von deutlich mehr als 220 °C oder bei Anzeigen mit aktiver Matrix weisen die bisher eingesetzten Materialien Nachteile auf.

Ähnliche Verbindungen der Formel I mit X = $R^1$ sind aus EP 0 125 563 B1 bekannt. Die dort beschriebenen 4-Ring-Verbindungen induzieren jedoch in vielen flüssigkristallinen Medien störende smektische Phasen und/oder zeigen keine ausreichende Löslichkeit besonders bei tiefen Temperaturen. trans, trans-4-[2-p-(trans-4-propylcyclohexyl)-phenyl-ethyl]-4'-propylbicyclohexyl zeigt beispielsweise folgende Phasenübergangstemperaturen: C 60 $\delta$ 233 N 261 I.

Die Verbindungen der Formel I mit X = $R^1$ zeigen eine überraschend gute Löslichkeit bei -20 °C in vielen flüssigkristallinen Medien anderer Stoffklassen.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hohen Klärpunkten, hervorragender Nematogenität bis zu tiefen Temperaturen, hervorragender chemischer Stabilität, ausgeprägtem $\epsilon_\perp$ bei positiver dieelektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität und hohem spezifischen Widerstand.

Als weitere Vorteile der Verbindungen der Formel I sind zu nennen:

- geringe Tendenz zur Ausbildung smektischer Phasen in Kombination mit nematischen Materialien mit

positiver dielektrischer Anisotropie,
- hohe Löslichkeit in nematischen Materialien mit positiver dielektrischer Anisotropie,
- die Eigenschaft den Klärpunkt von Mischungen deutlich zu erhöhen, die bereits eine hochklärende Komponente enthalten,
- sehr geringe Temperaturabhängigkeit der Schwellenspannung,
- gute chemische und photochemische Stabilität.

Diese herausragende Kombinatin von Eigenschaften ermöglicht die Bereitstellung von flüssigkristallinen Medien mit deutlich verbesserten Eigenschaften.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben n, $R^1$, A, X, Y und Z die angegebene Bedeutung, sofern nicht ausdrücklich etwas anderes vermerkt ist.

In den Verbindungen der Formel I bedeutet $R^1$ vorzugsweise $C_nH_{2n+1}$. Die Alkylgruppen $C_nH_{2n+1}$ sind vorzugsweise geradkettig. Dementsprechend bedeutet $C_nH_{2n+1}$ Methyl, Ethyl, n-Propyl, n-Butyl, n-Pentyl, n-Hexyl oder n-Heptyl. n ist vorzugsweise 2, 3, 4 oder 5.

Oxaalkyl ist vorzugsweise geradkettiges Alkoxy mit bis zu 7 C-Atomen oder 2-Oxapropyl (= Methoxymethyl), 2- (= Ethoxymethyl) oder 3-Oxybutyl (= 2-Methoxyethyl), 2-, 3- oder 4-Oxapentyl, 2-, 3-, 4- oder 5-Oxahexyl, 2-, 3-, 4-, 5- oder 6-Oxaheptyl oder 2-, 3-, 4-, 5-, 6- oder 7-Oxaoctyl.

Im Falle X = $R^1$ ist Ring A vorzugsweise trans-1,4-Cyclohexylen;

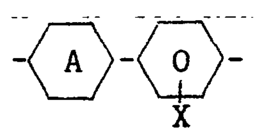

ist vorzugsweise eines der Ringsysteme 1 bis 5:

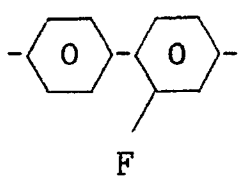

1

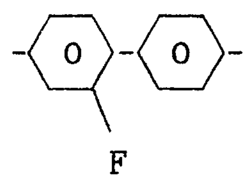

2

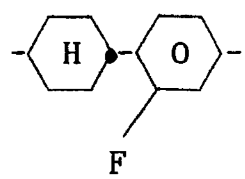

3

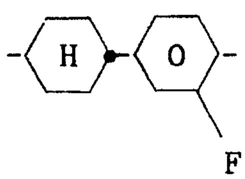

4

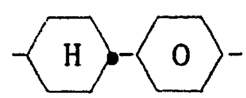

5

wovon 1, 2 und 4 besonders bevorzugt sind.

Verbindungen der Formel I mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl), 2-Methyl-butyl, Isopentyl (= 3-Methylbutyl), 2-Methylpentyl, 3-Methylpentyl oder 2-Heptyl (= 1-Methylhexyl).

Der Rest 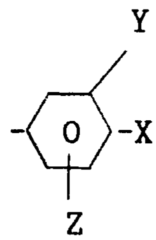 ist vorzugsweise

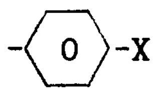, 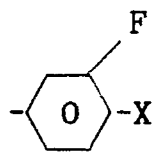 oder 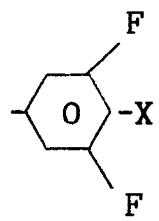.

X ist vorzugsweise F, Cl, $-CF_3$ oder $-OCF_3$.

Besonders bevorzugt sind die Verbindungen der folgenden Teilformeln:

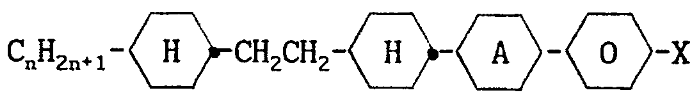

Ia

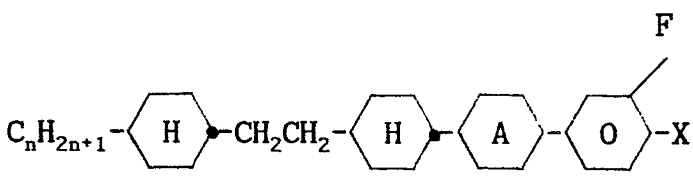

Ib

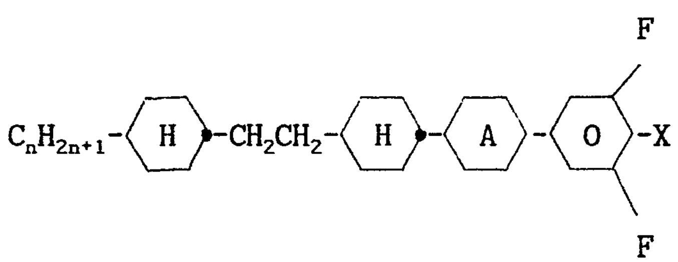

Ic

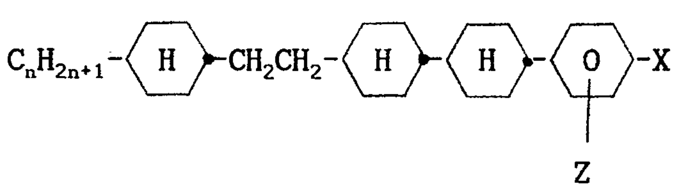

Id

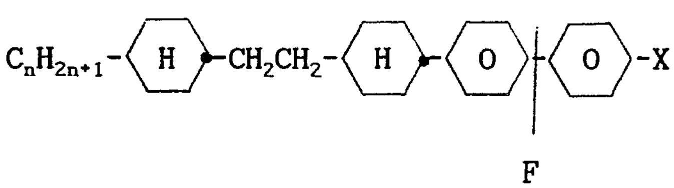

Ie

worin n, A, Z und X die angegebene Bedeutung haben.

Im Falle X = $R^1$ sind besonders die Verbindungen der folgenden Teilformeln bevorzugt, worin $R^2$ eine der Bedeutungen von $R^1$ hat und

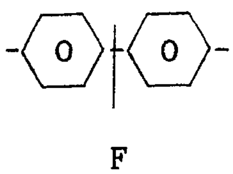

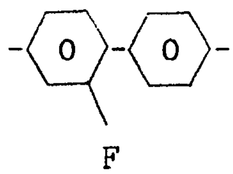

oder

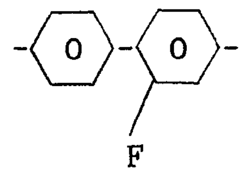

ist:

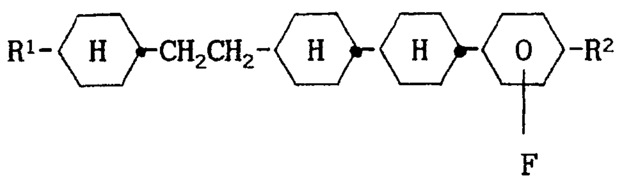

Ia′

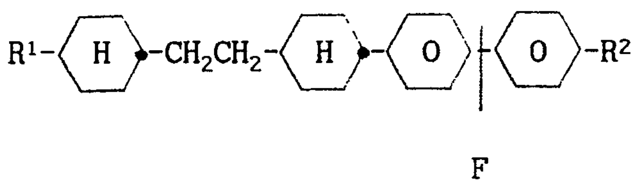

Ib′

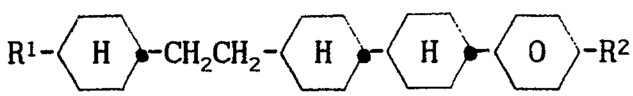

Ic′

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I können beispielsweise durch Umsetzung eines Aldehyds der Formel II

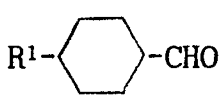

II

mit dem Phosphoniumsalz z.B. eines entsprechenden trans-4-(subst. Biphenyl)-cyclohexylmethylbromids nach Wittig und katalytischer Hydrierung, z.B. an Pd/C der erhaltenen Ethenderivate, erhalten werden. Umgekehrt ist natürlich auch die Umsetzung eines trans-4-$R^1$-Cyclohexylmethylphosphoniumsalzes mit einem Cyclohexylcarbaldehyd der Formel

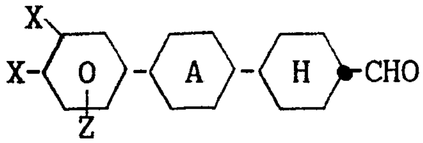

möglich. Letzeres Vorprodukt ist durch Oxidation des Cyclohexylmethylalkohols aus nachfolgendem Schema nach Standardmethoden erhältlich.

Zur Synthese der Cyclohexylmethylbromide bzw. Cyclohexylcarbaldehyde geeignete Vorstufen sind beispielsweise nach folgendem Syntheseschema erhältlich:

Schema 1

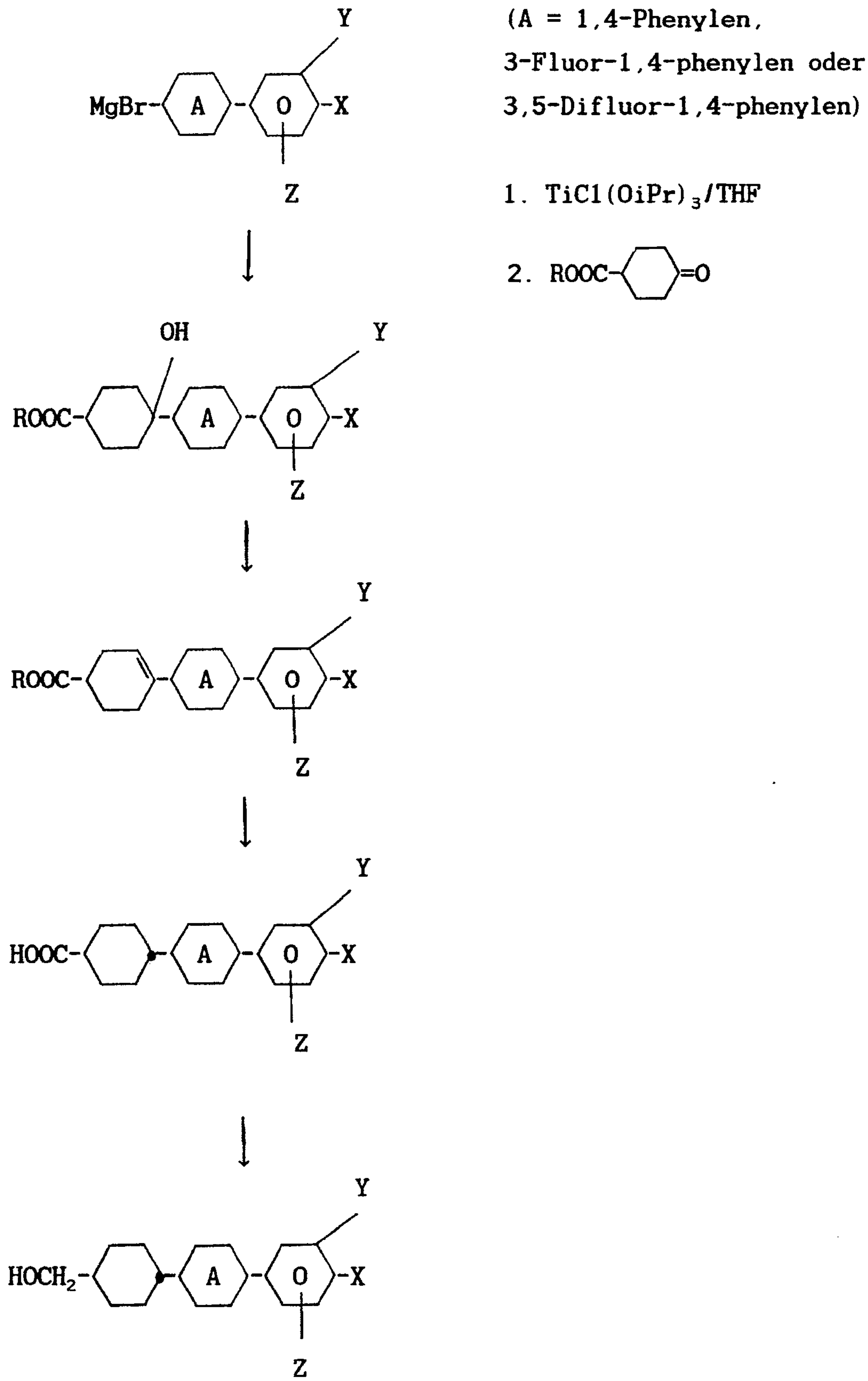


Die aus dem entsprechenden Brombiphenyl-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung, Isomerisierung und Reduktion zum Cyclohexylmethylalkohol erhöht man die geeignete Vorstufe.

Die als Ausgangsstoffe verwendete Brombiphenylderivate sind zum Teil bekannt, zum Teil können sie ohne Schwierigkeiten nach Standardverfahren der organischen Chemie aus literaturbedingten Verbindungen hergestellt werden. Beispielsweise sind die $OCF_3$- oder $OCHF_2$-Verbindungen nach bekannten Verfahren aus den entsprechenden Phenolen bzw. die $CF_3$- oder CN-Verbindungen aus den entsprechenden Benzoesäuren erhältlich. Verbindungen der Formel

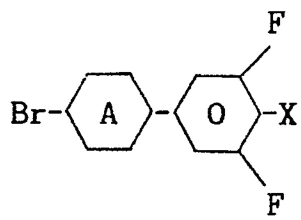

oder auch entsprechende monofluorierte Verbindungen sind beispielsweise aus den bekannten Vorstufen mit X = H durch Lithiierung bei tiefen Temperaturen und anschließende Umsetzung mit einem geeigneten Elektrophil erhältlich.

Weitere Synthesevarianten sind dem Fachmann bekannt. Bevorzugte Varianten sind dem Schema 2 zu entnehmen. Alle Ausgangsmaterialien sind entweder bekannt oder können in Analogie zu bekannten Verbindungen hergestellt werden (z.B. DE-OS 33 17 597, E. Poetsch, Kontakte/Darmstadt 1988 (2), S. 15-28).

Schema 2

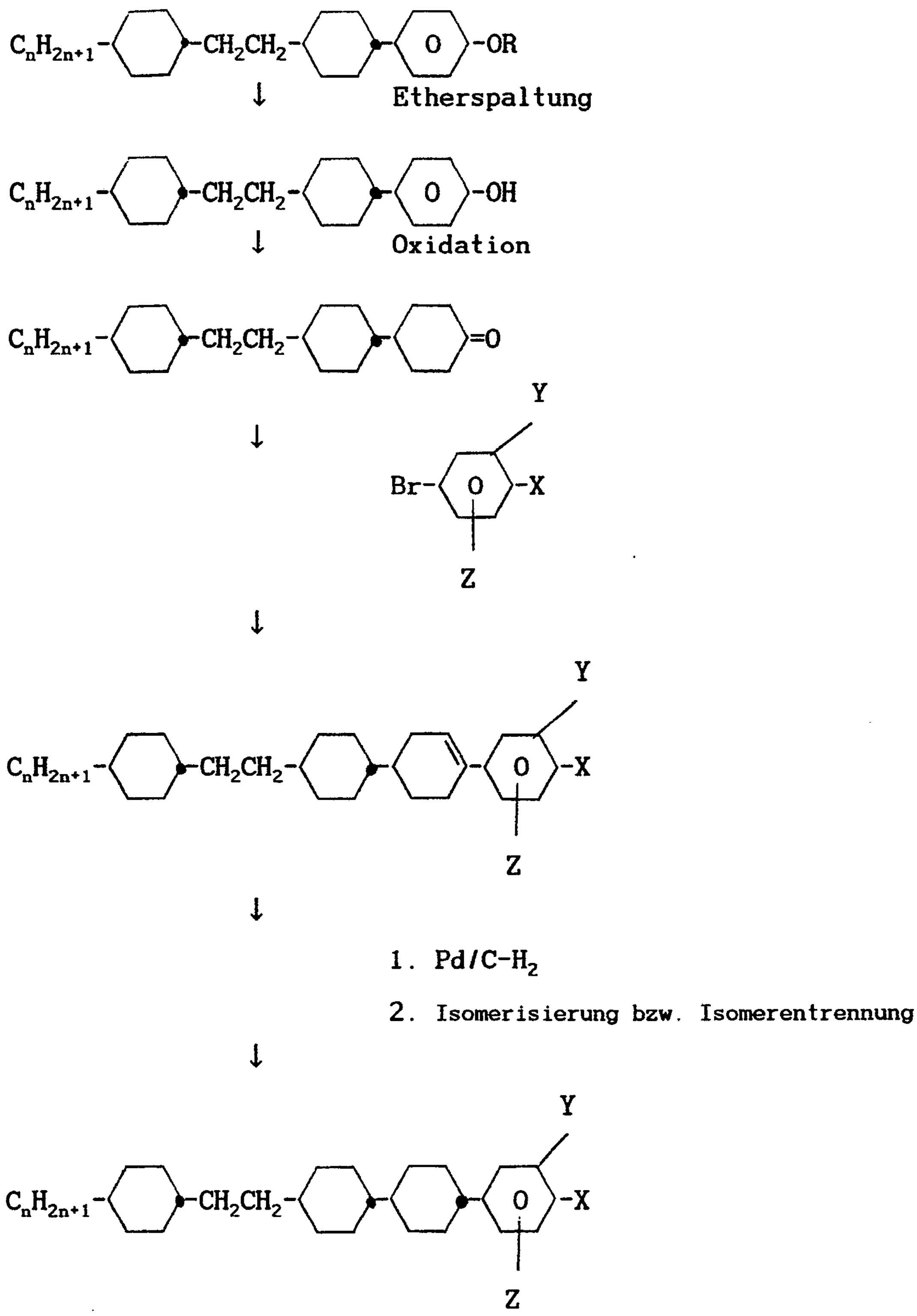


Einige ganz besonders bevorzugte Synthesevarianten sind den folgenden Schemata zu entnehmen:

Schema 3

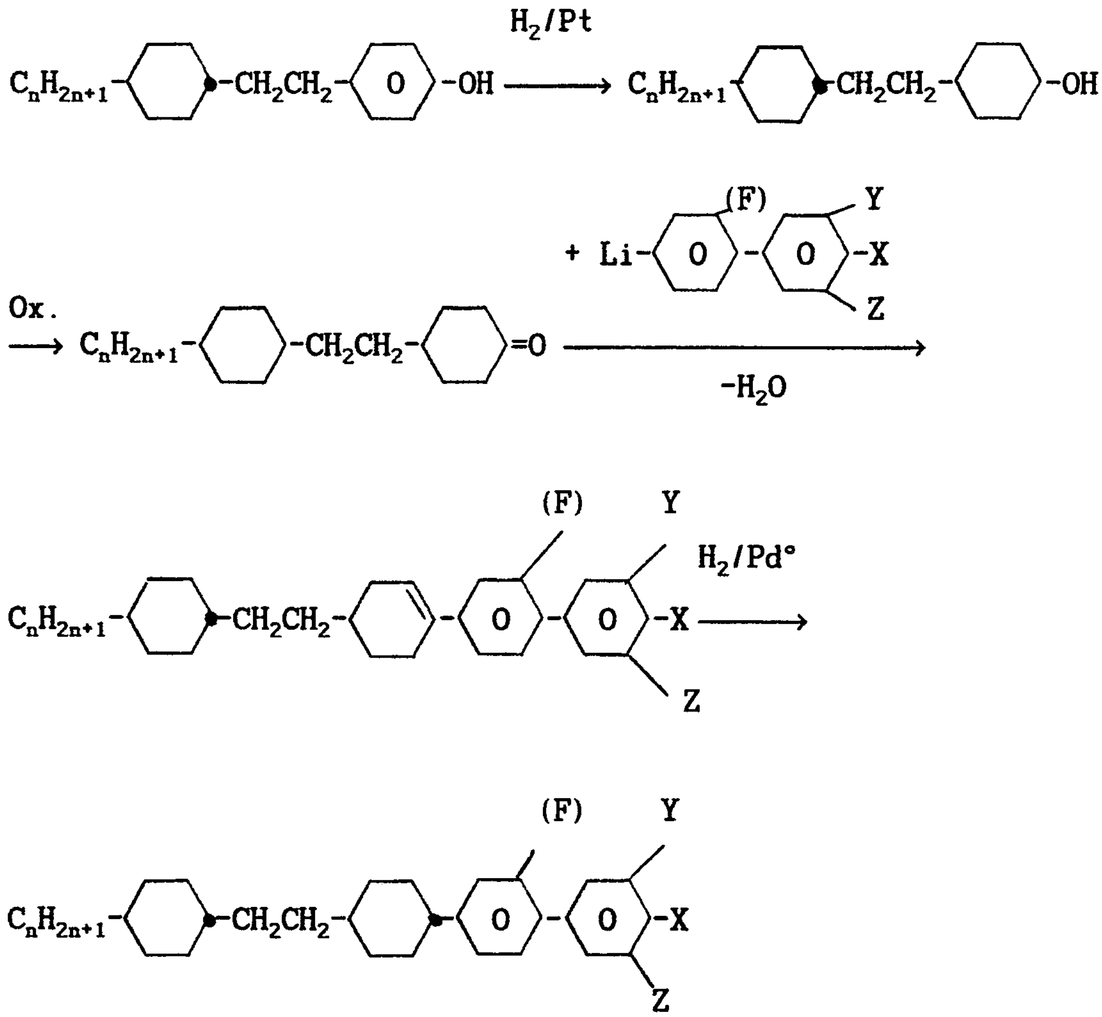


Schema 4

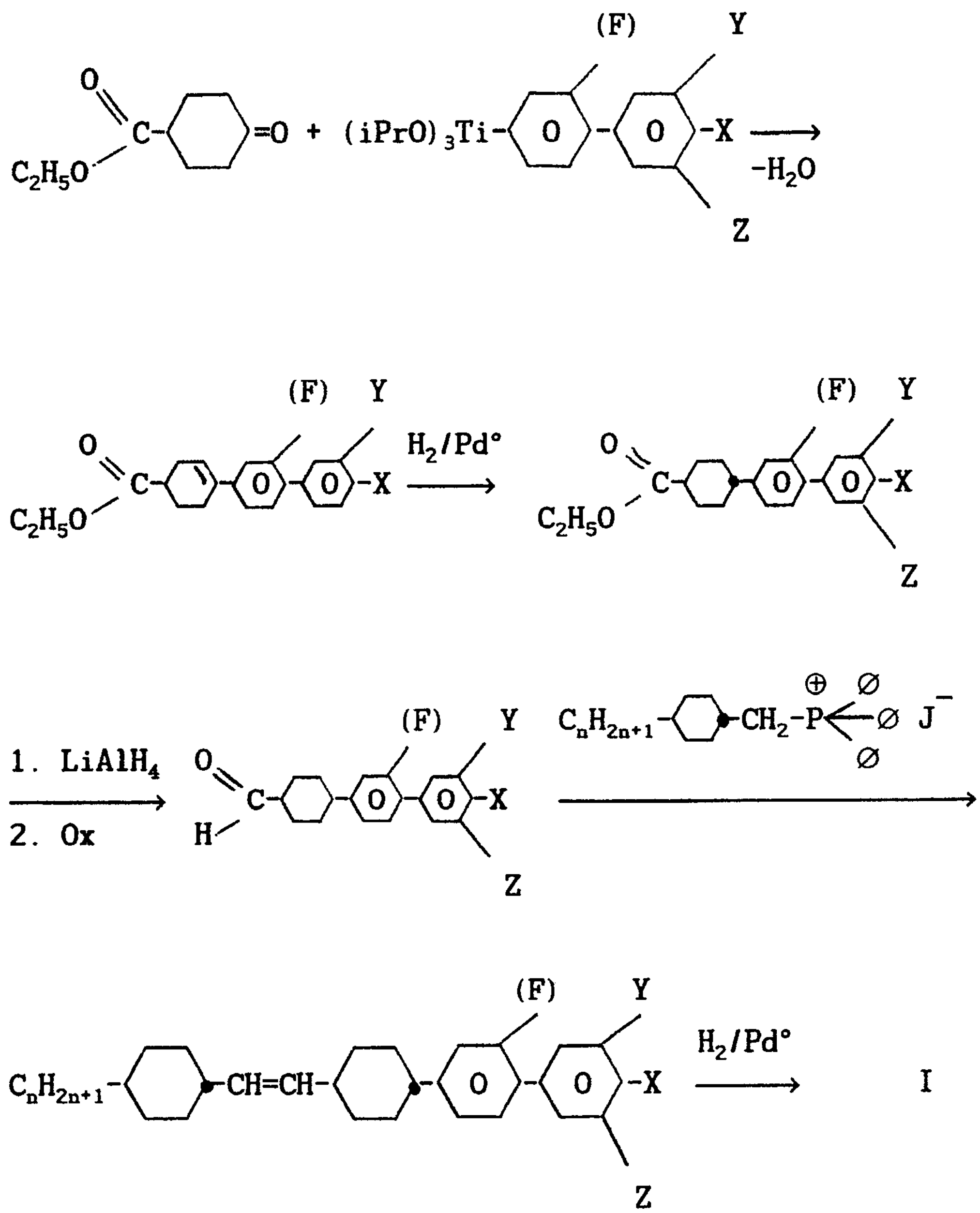


(Der Fluorsubstituent in Schema 3 und 4 in Klammer kann auch H bedeuten.)

Schema 5

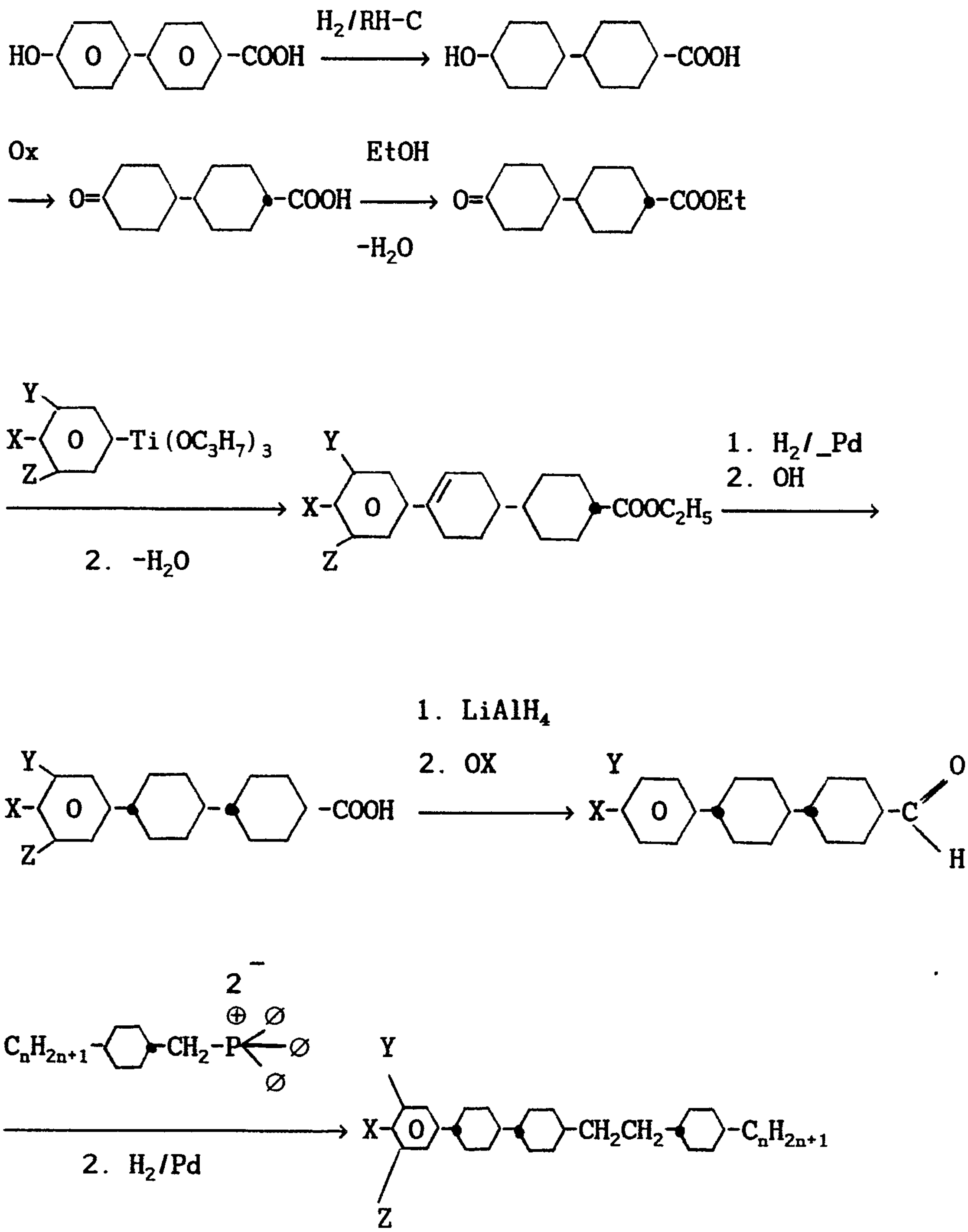


Schema 6

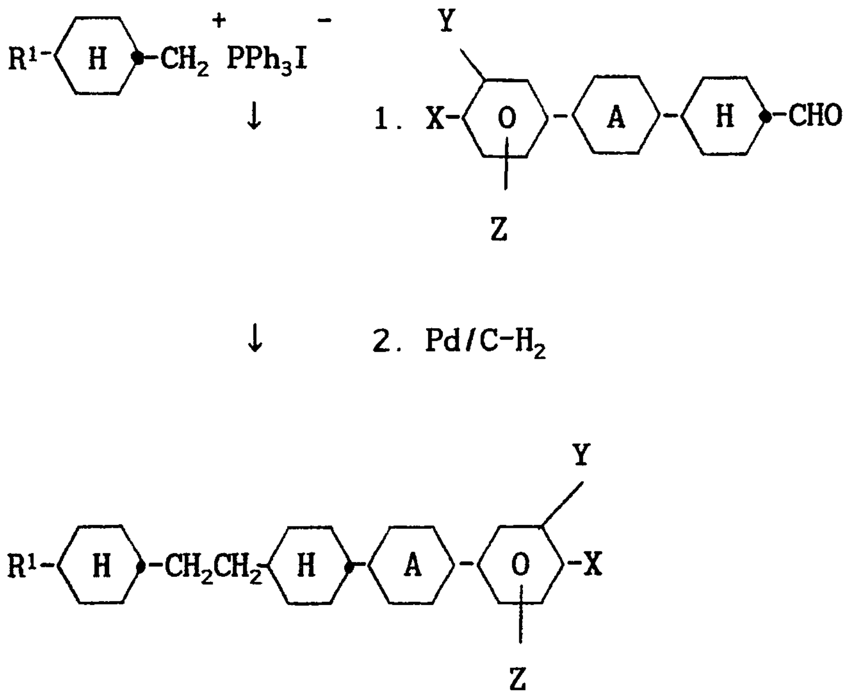


Schema 7

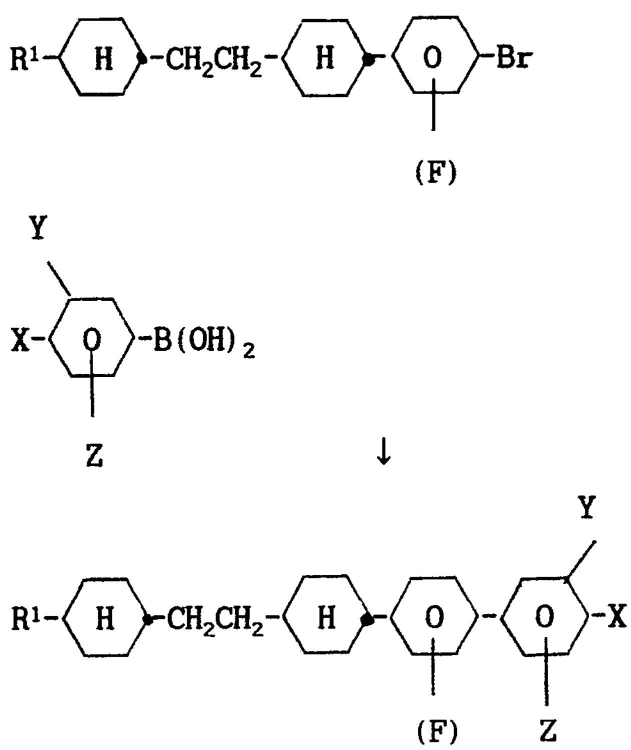

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausge~ wählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexan-carbonsäurephenyl-

oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1
R'-L-COO-E-R" 2
R'-L-OOC-E-R" 3
R'-L-$CH_2CH_2$-E-R" 4
R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor sub~ stituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten. Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Ver~ bindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, -$CF_3$, -$OCF_3$, F, Cl oder -NCS; R hat dabei die bei den Verbindungen der Teilformeln 1a bis 5a angegebene Bedeutung und ist vorzugsweise Alkyl oder Alkenyl. Besonders bevorzugt ist R" ausgewählt aus der Gruppe bestehend aus -F, Cl, $CF_3$ und -$OCF_3$. Aber auch andere Varianten der vorgesehenen Substituenten in den Verbin~ dungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:

Gruppe 1: 20 bis 90 %, insbesondere 30 bis 90 %,
Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTSOH | p-Toluolsulfonsäure |

Beispiel 1

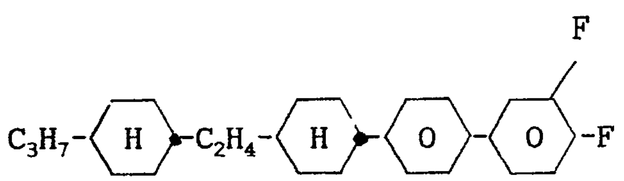

Das gemäß Schema 4 erhaltene 1-(trans-4-Propylcyclohexyl)-2-trans-4-(3,4-difluorbiphenyl-4'-yl)-cyclohexyl-ethen wird in an sich bekannter Weise am Pd-C bei Raumtemperatur zum Zielprodukt hydriert.

Beispiel 2 bis 64

Analog Beispiel 1 erhält man die folgenden Verbindungen:

| | n | X | Y | Z | A |
|---|---|---|---|---|---|
| (2) | 2 | $-CF_3$ | H | H | Ph |
| (3) | 5 | $-CF_3$ | H | H | Ph |
| (4) | 4 | $-CF_3$ | H | H | Ph |
| (5) | 5 | F | F | F | Ph |
| (6) | 2 | F | F | F | Ph |
| (7) | 3 | F | F | F | Ph |
| (8) | 5 | Cl | F | F | Ph |
| (9) | 2 | Cl | F | F | Ph |
| (10) | 3 | Cl | F | F | Ph |
| (11) | 5 | $-OCF_3$ | H | H | Ph |
| (12) | 2 | $-OCF_3$ | H | H | Ph |
| (13) | 3 | $-OCF_3$ | H | H | Ph |
| (14) | 5 | $-OCHF_2$ | H | H | Ph |
| (15) | 2 | $-OCHF_2$ | H | H | Ph |
| (16) | 3 | $-OCHF_2$ | H | H | Ph |
| (17) | 2 | F | H | H | Cy |
| (18) | 3 | F | H | H | Cy |
| (19) | 5 | F | H | H | Cy |
| (20) | 2 | Cl | H | H | Cy |
| (21) | 3 | Cl | H | H | Cy |
| (22) | 5 | Cl | H | H | Cy |
| (23) | 2 | F | F | H | Cy |
| (24) | 3 | F | F | H | Cy |
| (25) | 5 | F | F | H | Cy |

| | n | X | Y | Z | A |
|---|---|---|---|---|---|
| (26) | 2 | Cl | F | H | Cy |
| (27) | 3 | Cl | F | H | Cy |
| (28) | 5 | Cl | F | H | Cy |
| (29) | 2 | $-CF_3$ | H | H | Cy |
| (30) | 3 | $-CF_3$ | H | H | Cy |
| (31) | 5 | $-CF_3$ | H | H | Cy |
| (32) | 3 | $-CF_3$ | F | H | Cy |
| (33) | 3 | $-CF_3$ | F | H | Cy |
| (34) | 5 | $-CF_3$ | F | H | Cy |
| (35) | 2 | -CN | H | H | Cy |
| (36) | 3 | -CN | H | H | Cy |
| (37) | 5 | -CN | H | H | Cy |
| (38) | 2 | -CN | F | H | Cy |
| (39) | 3 | -CN | F | H | Cy |
| (40) | 5 | -CN | F | H | Cy |
| (41) | 2 | -CN | F | F* | Cy |
| (42) | 3 | -CN | F | F* | Cy |
| (43) | 5 | -CN | F | F* | Cy |
| (44) | 2 | $-OCF_3$ | H | H | Cy |
| (45) | 3 | $-OCF_3$ | H | H | Cy |
| (46) | 5 | $-OCF_3$ | H | H | Cy |
| (47) | 2 | $-OCHF_2$ | H | H | Cy |
| (48) | 3 | $-OCHF_2$ | H | H | Cy |
| (49) | 5 | $-OCHF_2$ | H | H | Cy |
| (50) | 2 | F | F | H | PhF |
| (51) | 3 | F | F | H | PhF |
| (52) | 5 | F | F | H | PhF |

| | n | X | Y | Z | A |
|---|---|---|---|---|---|
| (53) | 2 | Cl | F | H | PhF |
| (54) | 3 | Cl | F | H | PhF |
| (55) | 5 | Cl | F | H | PhF |
| (56) | 2 | -CN | H | H | PhF |
| (57) | 3 | -CN | H | H | PhF |
| (58) | 5 | -CN | H | H | PhF |
| (59) | 2 | $-CF_3$ | H | H | PhF |
| (60) | 3 | $-CF_3$ | H | H | PhF |
| (61) | 5 | $-CF_3$ | H | H | PhF |
| (62) | 2 | $-OCF_3$ | H | H | PhF |
| (63) | 3 | $-OCF_3$ | H | H | PhF |
| (64) | 5 | $-OCF_3$ | H | H | PhF |

* Z in ortho-Position zu X (Ph = 1,4-Phenylen, Cy = trans-1,4-Cyclohexylen, PhF = 3-Fluor-1,4-phenylen)

Beispiel 65

Gemäß Schema 6 erhaltenes 1-(trans-4-n-Propylcyclohexyl)-2-[trans-4-(2-fluor-4-n-propylbiphenyl-4'-yl)-cyclohexyl)-ethen wird bei Normaldruck an Pd/C hydriert. Nach üblicher Aufarbeitung erhält man 1-(trans-4-n-Propylcyclohexyl)-2-[trans-4-(2-fluor-4-n-propylbiphenyl-4'-yl)-cyclohexyl]-ethan.

Beispiele 66 bis 80

Die folgenden Verbindungen werden analog bzw. nach den obigen Schemata hergestellt:

| | $R^1$ | X | A | 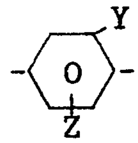 |
|---|---|---|---|---|
| (66) | ethyl | n-propyl | 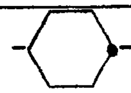 | 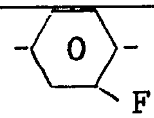 |
| (67) | n-pentyl | n-propyl | 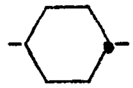 | 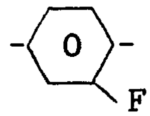 |
| (68) | n-propyl | ethyl | 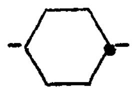 | 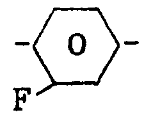 |
| (69) | n-propyl | n-pentyl | 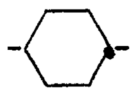 | 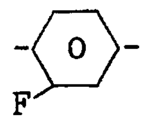 |
| (70) | ethyl | n-propyl | 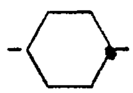 | 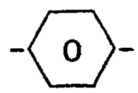 |
| (71) | n-propyl | ethyl | 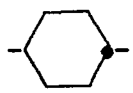 | 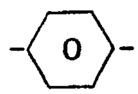 |
| (72) | n-pentyl | ethyl | 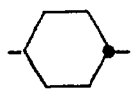 | 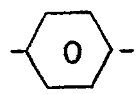 |
| (73) | n-pentyl | n-propyl | 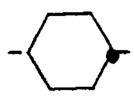 | 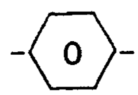 |
| (74) | n-propyl | n-propyl | 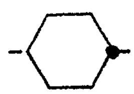 | 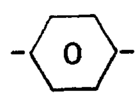 |

| | $R^1$ | X | A | 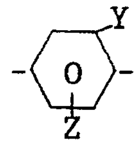 |
|---|---|---|---|---|
| (75) | n-propyl | n-propyl | 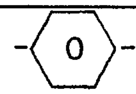 | 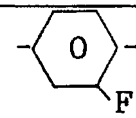 |
| (76) | n-propyl | n-propyl | 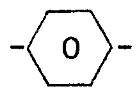 | 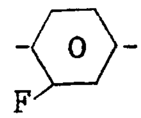 |
| (77) | ethyl | n-propyl | 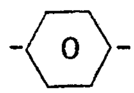 | 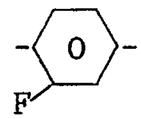 |
| (78) | n-propyl | ethyl | 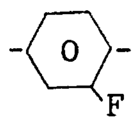 | 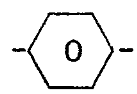 |
| (79) | n-pentyl | ethyl | 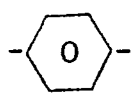 | 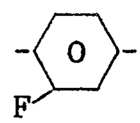 |
| (80) | n-pentyl | n-propyl | 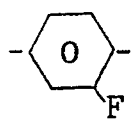 | 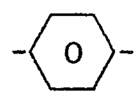 |

**Patentansprüche**

1. Phenylcyclohexylethane der Formel I

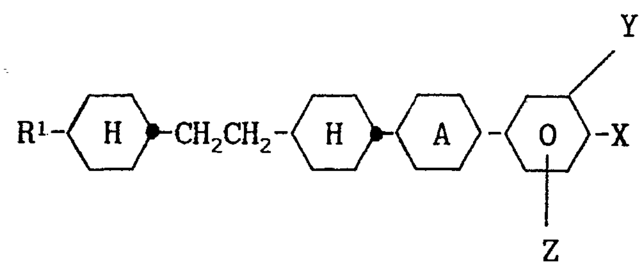

I

worin $R^1$ Alkyl oder Oxaalkyl mit bis zu 9 C-Atomen, Ring A trans-1,4-Cyclohexylen, 1,4-Phenylen, 3-Fluor-1,4-phenylen oder 3,5-Difluor-1,4-phenylen, X eine der Bedeutungen von $R^1$, F, Cl, $-CF_3$, -CN, $-OCF_3$ oder $-OCHF_2$ und Y und Z jeweils unabhängig voneinander H oder F bedeuten.

2. Phenylcyclohexylethane nach Anspruch 1, gekennzeichnet durch die Formel Ia

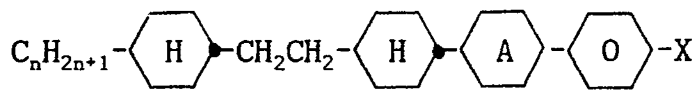

Ia

worin n 1 bis 7 und A und X die angegebene Bedeutung haben.

3. Phenylcyclohexylethane nach Anspruch 1, gekennzeichnet durch die Formel Ib

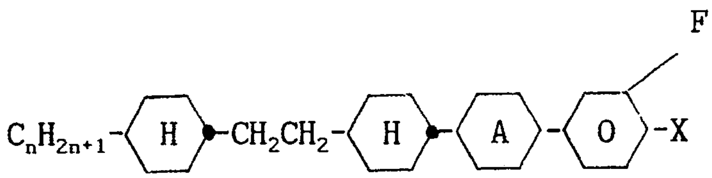

Ib

worin n 1 bis 7 und A und X die angegebene Bedeutung haben.

4. Phenylcyclohexylethane nach Anspruch 1, gekennzeichnet durch die Formel Ic

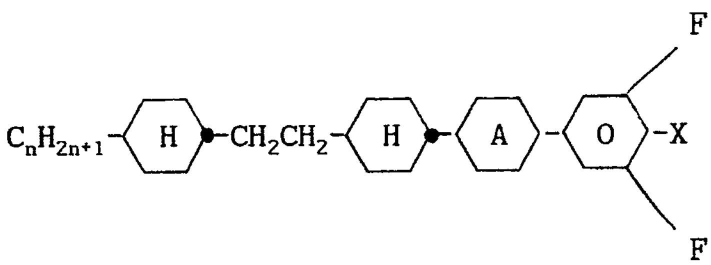

Ic

worin n 1 bis 7 und A und X die angegebene Bedeutung haben.

5. Phenylcyclohexylethane nach Anspruch 1, gekennzeichnet durch die Formel Id

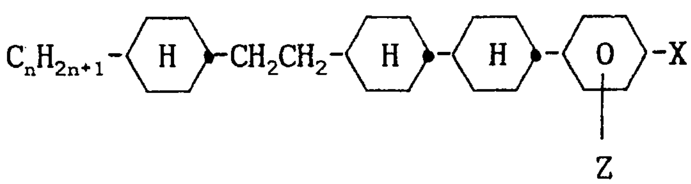

Id

worin n 1 bis 7 und X und Z die angegebene Bedeutung haben.

6. Phenylcyclohexylethane nach Anspruch 1, gekennzeichnet durch die Formel Ie

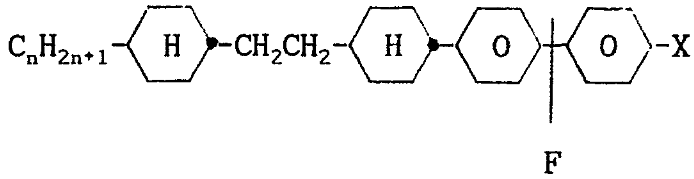

Ie

worin n 1 bis 7, 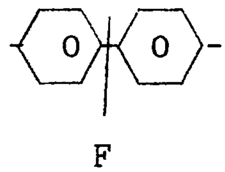

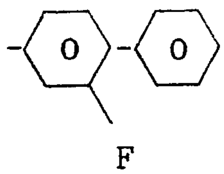 oder 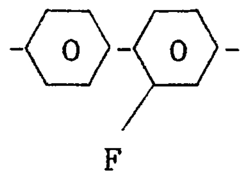 und

X die angegebene Bedeutung hat.

7. Phenylcyclohexylethane nach mindestens einem der Ansprüche 1 bis 4, dadurch gekennzeichnet, daß Ring A 1,4-Phenylen, 3-Fluor-1,4-phenylen oder 3,5-Difluor-1,4-phenylen bedeutet.

8. Verwendung der Phenylcyclohexylethane der Formel I nach Anspruch 1 als Komponenten flüssigkristalliner Medien für elektrooptische Anzeigen.

9. Flüssigkristallines Medium für elektrooptische Anzeigen mit mindestens zwei flüssigkristallinen Komponenten, dadurch gekennzeichnet, daß mindestens eine Komponente ein Phenylcyclohexylethan der Formel I nach Anspruch 1 ist.

10. Elektrooptische Anzeige auf der Basis einer Flüssigkristallzelle, dadurch gekennzeichnet, daß die Flüssigkristallzelle ein Medium nach Anspruch 9 enthält.